# EUROPEAN PATENT SPECIFICATION

(11) **EP 2 427 576 B1**
(45) Date of publication and mention of the grant of the patent: **22.06.2016**
(21) Application number: 10727839.2
(22) Date of filing: 10.05.2010
(51) Int. Cl.: C12Q 1/70

(54) **GENERIC ASSAYS FOR DETECTION OF INFLUENZA VIRUSES**
ALLGEMEINE UNTERSUCHUNGSVERFAHREN ZUM NACHWEIS VON INFLUENZA-VIRUS
DOSAGES GÉNÉRIQUES DE DÉTECTION DES VIRUS DE LA GRIPPE

(30) Priority: 08.05.2009 US 215704 P; 26.05.2009 US 217045 P
(43) Date of publication of application: 14.03.2012
(60) Divisional application: 16169958.2
(73) Proprietor: Seqirus UK Limited, London EC4V 6JA (GB)
(72) Inventor: ROTH, Bernhard, 35041 Marburg (DE)
(74) Representative: Wise, Daniel Joseph
(86) International application number: PCT/IB2010/001158
(87) International publication number: WO 2010/128396

(56) References cited:
- WO-A1-2005/012572
- WO-A1-2006/102695
- WO-A1-2008/140513
- WO-A2-2004/057021
- WO-A2-2007/095155
- WO-A2-2008/068631
- US-A1- 2009 081 648
- STONE BELINDA ET AL: "Rapid detection and simultaneous subtype differentiation of influenza A viruses by real time PCR." JOURNAL OF VIROLOGICAL METHODS MAY 2004 LNKD- PUBMED:15041206, vol. 117, no. 2, May 2004 (2004-05), pages 103-112, XP002604452 ISSN: 0166-0934
- DI TRANI LIVIA ET AL: "A sensitive one-step real-time PCR for detection of avian influenza viruses using a MGB probe and an internal positive control" BMC INFECTIOUS DISEASES, BIOMED CENTRAL, LONDON, GB LNKD- DOI:10.1186/1471-2334-6-87, vol. 6, no. 1, 25 May 2006 (2006-05-25), page 87, XP021015749 ISSN: 1471-2334
- VAN ELDEN L J R ET AL: "Simultaneous detection of influenza viruses A and B using real-time quantitative PCR" JOURNAL OF CLINICAL MICROBIOLOGY, vol. 39, no. 1, January 2001 (2001-01), pages 196-200, XP002604453 ISSN: 0095-1137
- SPACKMAN ERICA ET AL: "Development of a real-time reverse transcriptase PCR assay for type A influenza virus and the avian H5 and H7 hemagglutinin subtypes" JOURNAL OF CLINICAL MICROBIOLOGY, AMERICAN SOCIETY FOR MICROBIOLOGY, WASHINGTON, DC, US LNKD- DOI:10.1128/JCM.40.9.3256-3260.2002, vol. 40, no. 9, 1 September 2002 (2002-09-01), pages 3256-3260, XP002473651 ISSN: 0095-1137
- FOUCHIER R A M ET AL: "Detection of influenza A viruses from different species by PCR amplification of conserved sequences in the matrix gene" JOURNAL OF CLINICAL MICROBIOLOGY, AMERICAN SOCIETY FOR MICROBIOLOGY, WASHINGTON, DC, US, vol. 38, no. 11, 1 November 2000 (2000-11-01), pages 4096-4101, XP002555829 ISSN: 0095-1137
- KISS I ET AL: "Application of real-time RT-PCR utilising lux (light upon extension) fluorogenic primer for the rapid detection of avian influenza viruses" ACTA VETERINARIA HUNGARICA, vol. 54, no. 4, 2006, pages 525-533, XP002604454 ISSN: 0236-6290
- LANDOLT GABRIELE A ET AL: "Use of real-time reverse transcriptase polymerase chain reaction assay and cell culture methods for detection of swine influenza A viruses." AMERICAN JOURNAL OF VETERINARY RESEARCH JAN 2005 LNKD- PUBMED:15691046, vol. 66, no. 1, January 2005 (2005-01), pages 119-124, XP009139695 ISSN: 0002-9645
- STONE BELINDA ET AL: "Rapid detection and simultaneous subtype differentiation of influenza A viruses by real time PCR", JOURNAL OF VIROLOGICAL METHODS, ELSEVIER BV, NL, vol. 117, no. 2, 1 May 2004 (2004-05-01), pages 103-112, XP002457249, ISSN: 0166-0934, DOI: 10.1016/J.JVIROMET.2003.12.005

## Description

This patent application claims priority from United States provisional patent application 61/215,704, filed 8th May 2009, and United States provisional patent application 61/217,045, filed 26th May 2009.

### TECHNICAL FIELD

The present invention relates to novel, generic methods for the detection and quantification of influenza viruses. The invention preferably uses a reverse transcription (RT-PCR) Real Time (q-PCR) assay which amplifies a conserved region within influenza A or B strains. The inventive assays allow the quantification of influenza virus RNA molecules or whole virus particles, irrespective of the particular virus strain (e.g. human, avian, swine flu). The inventive methods are particularly applicable as diagnostic assays or in the monitoring of vaccine production processes.

### BACKGROUND OF THE INVENTION

Various forms of influenza vaccines are currently available. Vaccines are generally based either on live virus or on inactivated virus. Inactivated vaccines may be based on whole virions, 'split' virions, or on purified surface antigens (see details in WO 2008/068631 to which is expressly referred). Influenza vaccines are typically trivalent and contain two influenza A strains and one influenza B strain. Besides the traditional egg-based production methods for influenza vaccines, different cell culture based manufacturing methods have been described more recently (*e.g.* see chapters 17 and 18 in: Vaccines, eds. Plotkin & Orenstein; 4th edition, 2004, ISBN: 0-7216-9688-0; Wilschut; Mc Elhaney, Palache in "Influenza"; 2. Edition; Elsevier 2006; ISBN 0-7234-3433-6 Chapter 9).

The application of nucleic acid based detection methods within the influenza vaccine production process (*e.g.* for quality control processes) has so far been limited. This is due to the fact that the influenza strains used in vaccine production change from season to season, and that thus for every season a new, strain specific detection assay would need to be developed. The present invention provides a novel nucleic acid assay analyzing a conserved region within the genome of influenza A or influenza B strains (irrespective of origin, *e.g.* human, avian, swine flu). These assays are therefore suitable for analyzing a variety of influenza strains.

The invention provides a method for quantifying the amount of intact influenza virus particles in a sample, characterised in that the following steps are conducted:
a) free virus RNA is removed from the sample,
b) a method for detecting the remaining influenza virus RNA in the sample is applied, characterised in that a conserved region within the influenza genome is amplified,
c) the signal generated in part (b) is compared to the signal generated by a standard RNA,
d) the amount of intact virus particles is concluded.

The invention also provides a method for the production of influenza vaccines, in which a method of the invention for quantifying the amount of intact influenza virus particles in a sample is applied within the fermentation step of a cell culture based production process, and that the amount of virus particle is quantified in order to determine the optimal time for harvesting the viruses.

The invention also provides a method for the cell culture-based production of an influenza vaccine, characterised in that the following steps are conducted:
- cells are propagated in a fermentation vessel;
- seed viruses are added;
- the virus propagation is monitored using the method of the invention for quantifying the amount of intact influenza virus particles in a sample;
- the virus suspension is centrifuged and filtered;
- the virus is purified by chromatography and ultra-/diafiltration steps, inactivated, disrupted to solubilize the viral surface antigens HA and NA;
- the antigens are filtrated to obtain monovalent bulk; and
- optionally, blending the monovalent bulk into multivalent bulks (typically trivalent bulks) and filling into final container.

### DISCLOSURE OF THE INVENTION

The present invention describes a novel method for detecting influenza virus RNA. The inventive methods analyse a conserved region within the influenza A or influenza B virus genome, preferably the region encoding the matrix (M) protein. The M gene nucleotide sequences from GenBank which were used for an alignment of the influenza A M genes and the influenza B M genes are shown in tables 3 and 4, respectively.

For the analysis, a nucleic acid assay is conducted. A preferred assay is Reverse Transcriptase Polymerase Chain Reaction (RT-PCR). However, equivalent RNA amplification methods are also applicable, as known to the person skilled in the art (Nucleic Acid Sequence Based Amplification or NASBA™ as in US-5409818; 3SR™; Transcription Mediated Amplification or TMA™ as in US-5399491 *etc.).* The nucleic acid assay is preferably run as a real time assay (e.g. "qPCR"; Taqman™, Lightcycler™; Scorpion™ *etc*.).

### Detailed description of the preferred "one step RT-real time PCR"

In a particularly preferred embodiment, a one step RT-real time PCR assay is used ("one step RT-qPCR"). The person skilled in the art is familiar with conducting such "one step RT qPCR" assays. He knows how to find detailed reaction conditions for such amplification. Thus the reverse transcription reaction (RT) and the amplification reaction (qPCR) may be performed in the same vessel (*e.g.* in a single tube or vial) rather than in separate vessels.

Preferably, commercially available RT-PCR kits are used, e.g. Qiagen QuantiTect™ Virus kit or Invitrogen Super Script™ III Platinum™ kit. The generated fluorescence signals can be analyzed using the respective real time cycler software, as known in the art.

The inventive nucleic acid assays can be quantified by comparing the generated fluorescence signal with the respective signal of a standard nucleic acid, as known in the art. As such standard, a dilution series of an in vitro transcript (IVT) of the respective virus regions is preferably applied. Suitable IVTs can be generated as required or are commercially available *e.g.* Panomics™ supplies "Ifn-A" (282 nucleotides) and "Ifn-B" (276 nucleotides) single-stranded RNA molecules at 10ng/ml.

Preferably, RT-q PCR is performed using the primer and probe sequences shown in table 1 below. However, the person skilled in the art knows how to design additional, equivalent primers and probes directed to the virus genome encoding the M protein or to other conserved regions within the influenza genome. The person skilled in the art knows that the Taqman probes shown in the table below can be substituted by equivalent Lightcycler probes or other real time probe systems.

In a particular preferred embodiment, the primers of SEQ ID NO 4 and SEQ ID NO 7 are combined with the probe of SEQ ID NO 3 for the detection of Influenza virus A. In another preferred ' embodiment, the primers of SEQ ID NO 11 and SEQ ID NO 1 are combined with the probe of SEQ ID NO 9 for the detection of Influenza B viruses.

The examples (see below) show that the inventive one step RT qPCR assay is capable of detecting influenza viruses from different origins.

### Preferred embodiment: detection of intact viruses

In a particular preferred embodiment, the inventive assays are used to determine the amount of intact virus particles within a sample. This is particularly useful for monitoring vaccine production processes (see in detail below). A differentiation between free virus RNA or nucleoprotein-associated RNA and RNA within virus particles can be achieved by removing the free RNA from the sample prior to the amplification. This can be done, for example, by RNase treatment, as known in the art. A preferred process is as follows:
(a) take a a sample of virions, viral RNA and nucleoprotein (*e.g.* 21µl volume).
(b) incubate with RNase. For example, use a mixture of RNase A/T1 (15U/7.5U) for 1 hour).
(c) dilute the sample. For example, predilutions (1:100-1:10000) of sample are performed using a pipetting robot to get reliable quantitative results in the linear range of the qPCR (Cₜ 15-33);
(d) extract nucleic acid. For example, fully automated vRNA extraction by using magnetic beads;
(e) qPCR. qPCR can be set up by a pipetting robot. The calculation of copies/mL in the sample is in relation o a standard curve of UV-quantified *in vitro* transcribed RNA (IVT).

Thus the invention provides a method for quantifying the amount of intact influenza virus particles in a sample, comprising steps of: (a) removing non-virion-encapsulated RNA from the sample (*e.g*. by RNase digestion); (b) amplifying and quantifying remaining RNA in the sample (as disclosed herein); (c) using the results of step (b) to calculate the amount of intact virus particles in the sample. This method is particularly useful for influenza A and B viruses, especially during vaccine manufacture.

Step (b) may involve quantitative PCR (*e.g.* RT-PCR). The results of step (b) are compared to the signal generated by an standard RNA as part of the step (c) calculation. Between steps (a) and (b), virions may be treated to release their RNA, or this release may occur inherently as the PCR process is performed.

### Preferred application of the invention in influenza vaccine production

The inventive assays are particularly useful for egg-based or cell-culture based influenza vaccine production (for review see: Wilschut; Mc Elhaney, Palache in "Influenza"; 2. Edition; Elsevier 2006; ISBN 0-7234-3433-6 Chapter 9). The invention can be used at different steps during vaccine production, in particular in order to monitor and quantify virus yields in early process stages. The inventive process is in principle suitable for the production of various forms of influenza vaccines *(e.g.* live virus, inactivated whole virions, 'split' virions, purified surface antigens; for details see WO 2008/068631 to which applicant expressly refers). In these production methods, virions are grown in and harvested from virus containing fluids, *e.g.* allantoic fluid or cell culture supernatant. For the purification of the virions, different methods are applicable, *e.g.* zonal centrifugation using a linear sucrose gradient solution that includes detergent to disrupt the virions. Antigens may then be purified, after optional dilution, by diafiltration. Split virions are obtained by treating purified virions with detergents (*e.g.* ethyl-ether, polysorbate 80, deoxycholate, tri-N-butyl phosphate, Triton X-100, Triton N-101, cetyltrimethylammonium bromide, Tergitol NP9, etc.) to produce subvirion preparations, including the 'Tween-ether' splitting process. Methods of splitting influenza viruses, for example, are well known in the art (for review see WO 2008/068631). Examples of split influenza vaccines are the BEGRIVAC™, FLUARIX™, FLUZONE™ and FLUSHIELD™ products. The methods of the invention may also be used in the production of live vaccines. The viruses in these vaccines may be attenuated. Live virus vaccines include MedImmune's FLUMIST™ product (trivalent live virus vaccine). Purified influenza virus surface antigen vaccines comprise the surface antigens hemagglutinin and, typically, also neuraminidase. Processes for preparing these proteins in purified form are well known in the art. The FLUVIRIN™, AGRIPPAL™ and INFLUVAC™ products are influenza subunit vaccines. Another form of inactivated antigen is the virosome (nucleic acid free viral like liposomal particles). Virosomes can be prepared by solubilization of virus with a detergent followed by removal of the nucleocapsid and reconstitution of the membrane containing the viral glycoproteins. An alternative method for preparing virosomes involves adding viral membrane glycoproteins to excess amounts of phospholipids to give liposomes with viral proteins in their membrane.

### Particularly preferred application of the invention in cell culture based influenza vaccine production

In a particularly preferred embodiment, the invention is used in cell-culture based influenza vaccine production. Suitable cell lines are described *e.g.* in WO 2008/068631. The most preferred cell lines for growing influenza viruses are MDCK cell lines. The original MDCK cell line is available from the ATCC as CCL-34, but derivatives of this cell line and other MDCK cell lines may also be used. For instance, in WO97/37000 a MDCK cell line is disclosed that was adapted for growth in suspension culture ('MDCK 33016', deposited as DSM ACC 2219). Similarly, WO01/64846 discloses a MDCK-derived cell line that grows in suspension in serum-free culture ('B-702', deposited as FERM BP-7449). WO2006/071563 discloses non-tumorigenic MDCK cells, including 'MDCK-S' (ATCC PTA-6500), 'MDCK-SFlOl' (ATCC PTA-6501), 'MDCK-SF102' (ATCC PTA-6502) and 'MDCK-SF103' (PTA-6503). WO2005/113758 discloses MDCK cell lines with high susceptibility to infection, including 'MDCK.5F1' cells (ATCC CRL-12042). Any of these MDCK cell lines can be used.

The cell culture based vaccine production process usually comprises the following steps: The starting material for each monovalent bulk is a single vial of the MDCK working cell bank (WCB). The cells are propagated in a chemically defined medium to optimize cell growth during production. The WCB are expanded by sequential passage in spinner flasks followed by scale up in larger fermentation vessels. Seed virus is added and virus propagation in the fermenter is performed over a period of two to four days. At the end of the infection cycle, the virus suspension is centrifuged and filtered to remove residual intact cells from the culture harvest. The centrifuged, filtered bulk termed clarified virus harvest is the end of the fermentation process. The clarified virus harvest may be stored at room temperature (16-25°C) in a stainless steel storage vessel for up to 24 hours. The influenza virus is purified by chromatography and ultra-/diafiltration steps, inactivated by beta-propiolactone (BPL) and disrupted by cetyltrimethylammonium bromide (CTAB) to solubilize the viral surface antigens HA and NA. The drug substance production process concludes with a filtration of the concentrate into the final bulk vessel to obtain monovalent bulk. Finally, the monovalent bulks can be blended into multivalent bulks (typically trivalent bulks) and filled into their final container, e.g. syringes. It is standard practice to minimize the amount of residual cell line DNA in the final vaccine, in order to minimize any oncogenic activity of the DNA (see in detail WO 2008/068631).

The present invention can be applied at different points within this process. However, it is particularly preferred that the methods of the invention are used for the monitoring and/or quantifying of virus yields in the early process stages (fermentation, harvest, until inactivation). The inventive assays can be applied as supplemental or alternative methods to the state of the art method (Single radial diffusion (SRD)). The inventive method is rapid (results within ~4 hours; SRD ~3 days) and allows an application in high sample throughput. The method is independent from specific reagents (*e.g.* strain-specific antibodies). The inventive assay is particularly applicable where the sensitivity of SRD is too low (before purification/concentration) or SRD results are unreliable (at harvest / not virus particle associated HA is measured).

In a particularly preferred embodiment, the invention is used to quantify the virus load during the fermentation, in order to determine the optimal time for harvesting the viruses.

In a further particularly preferred embodiment, the nucleic acid analysis is preceded by a RNase digestion of the virus sample. It is such possible to distinguish between free virus RNA and intact virus particles, and thus to quantify the intact virus particles.

### Vaccine compositions and vaccine administration

The present disclosure also describes vaccines produced by the inventive manufacturing processes described above. Such vaccines typically contain HA as the main immunogen, and vaccine doses are standardised by reference to HA levels, typically measured by SRD. Existing vaccines typically contain about 15µg of HA per strain, although lower doses can be used *e.g.* for children, or in pandemic situations, or when using an adjuvant. Fractional doses such as ½ (*i.e.* 7-5µg HA per strain), ¼ and 1/8 have been used, as have higher doses *(e.g.* 3x or 9x doses). Thus vaccines may include between 0.1 and 150µg of HA per influenza strain, preferably between 0.1 and 50µg *e.g.* 0.1-20µg, 0.1-15µg, 0.1-10µg, 0.1-7.5µg, 0.5-5µg, *etc.* Particular doses include *e.g.* about 45, about 30, about 15, about 10, about 7.5, about 5, about 3.8, about 3.75, about 1.9, about 1.5, *etc.* per strain. For live vaccines, dosing is measured by median tissue culture infectious dose (TCID50) rather than HA content, and a TCID50 of between 10⁶ and 10⁸ (preferably between 10^{6.5}-10^{7.5}) per strain is typical.

Influenza strains produced with the invention may have a natural HA as found in wild-type viruses, or a modified HA. For instance, it is known to modify HA to remove determinants (*e.g.* hyper-basic regions around the HA1/HA2 cleavage site) that cause a virus to be highly pathogenic in avian species. The use of so called "reverse genetics" facilitates such modifications.

Influenza virus strains for use in vaccines change from season to season. In interpandemic periods, vaccines typically include two influenza A strains (H1N1 and H3N2) and one influenza B strain, and trivalent vaccines are typical. The invention may also be used in vaccine production against pandemic viral strains (*i.e.* strains to which the vaccine recipient and the general human population are immunologically naïve, in particular of influenza A virus), such as H2, H5, H7 or H9 subtype strains and also H1 subtype strains, and influenza vaccines for pandemic strains may be monovalent or may be based on a normal trivalent vaccine supplemented by a pandemic strain. Depending on the season and on the nature of the antigen included in the vaccine, however, the invention may also be used in vaccine production against one or more of HA subtypes H1, H2, H3, H4, H5, H6, H7, H8, H9, H10, H11, H12, H13, H14, H15 or H16, and/or NA subtypes N1, N2, N3, N4, N5, N6, N7, N8 or N9.

As well as being suitable for the production of vaccines against interpandemic strains, the compositions of the disclosure are particularly useful for the production of vaccines against pandemic strains. The characteristics of an influenza strain that give it the potential to cause a pandemic outbreak are: (a) it contains a new hemagglutinin compared to the hemagglutinins in currently-circulating human strains, *i.e.* one that has not been evident in the human population for over a decade *(e.g.* H2), or has not previously been seen at all in the human population *(e.g.* H5, H6 or H9, that have generally been found only in bird populations), such that the human population will be immunologically naïve to the strain's hemagglutinin; (b) it is capable of being transmitted horizontally in the human population; and (c) it is pathogenic to humans. A virus with H5 hemagglutinin type is preferred for immunizing against pandemic influenza, such as a H5N1 strain. Other possible strains include H5N3, H9N2, H2N2, H7N1 and H7N7, and any other emerging potentially pandemic strains and also H1N1 strains.

Compositions of the disclosure may include antigen(s) from one or more (e.g. 1, 2, 3, 4 or more) influenza virus strains, including influenza A virus and/or influenza B virus. Where a vaccine includes more than one strain of influenza, the different strains are typically grown separately and are mixed after the viruses have been harvested and antigens have been prepared. Thus a process of the invention may include the step of mixing antigens from more than one influenza strain. A trivalent vaccine is typical, including antigens from two influenza A virus strains and one influenza B virus strain. A tetravalent vaccine might also useful, including antigens from two influenza A virus strains and two influenza B virus strains, or three influenza A virus strains and one influenza B virus strain (WO2008/068631).

Vaccine compositions manufactured according to the invention are pharmaceutically acceptable. They usually include components in addition to the antigens e.g. they typically include one or more pharmaceutical carrier(s) and/or excipient(s). As described below, adjuvants may also be included. A thorough discussion of such components is available in reference (WO2008/068631 to which expressly is referred to). Compositions of the disclosure may advantageously include an adjuvant, which can function to enhance the immune responses (humoral and/or cellular) elicited in a patient who receives the composition. Preferred adjuvants comprise oil-in-water emulsions. Various such adjuvants are known, and they typically include at least one oil and at least one surfactant, with the oil(s) and surfactant(s) being biodegradable (metabolisable) and biocompatible. The oil may comprise squalene. The oil droplets in the emulsion are generally less than 5µm in diameter, and ideally have a sub-micron diameter, with these small sizes being achieved with a microfluidiser to provide stable emulsions. Droplets with a size of less than 220nm are preferred as they can be subjected to filter sterilization. Potential adjuvants are described in detail in WO2008/068631 (page 14 following; to which expressly is referred to; *e.g.* MF59™). Suitable containers for compositions of the present disclosure (or kit components) include sterile vials, syringes (*e.g.* disposable syringes), nasal sprays, etc. Such containers are described in detail in WO2008/068631 (page 31, to which expressly is referred to).

The disclosure describes a vaccine manufactured according to the invention. These vaccine compositions are suitable for administration to human patients, and the disclosure describes a method of raising an immune response in a patient, comprising the step of administering a composition of the invention to the patient (described in detail in WO 2008/068631; pages 32*ff*), to which expressly is referred to).

### Sequences and Kits

The disclosure describes the primer and probe sequences outlined in Table 1 (SEQ ID NOs 1, 2, 3, 4, 5, 6, 7, 8, 9, 10, and 11). SEQ ID NO: 8 includes two degenerate bases and so may be present as four separate and individual oligonucleotide sequences.

For influenza A: forwards primers include SEQ ID NOs: 5, 2 and 7; a reverse primer is SEQ ID NO: 4; and a useful probe is SEQ ID NO: 3. For influenza B: reverse primers include SEQ ID NOs: 8, 10 and 1; a forwards primer is SEQ ID NO: 11; and a useful probe is SEQ ID NO: 9. SEQ ID NO: 6 is a further forwards primer for influenza A. The term 'forwards' is used only for convenience and refers to a primer having the same sense as the ATG-containing coding strand for the matrix proteins. As influenza virus has a negative-stranded genome the terms forwards and reverse may be inverted when referring to hybridization to a viral genomic RNA segment.

The disclosure also describes the specific combination of the primers of SEQ ID NOs 4 and 7 with the probe of SEQ ID NO 3 (Influenza A), and the specific combination of the primers of SEQ ID NOs 11 and 1 with the probe of SEQ ID NO 9 (Influenza B), in particular in the form of a kit. The kit might contain further components, e.g. buffers, polymerases and further reaction components for the amplification. A further part of the present disclosure is the use of said sequences, combinations and kits for the detection of influenza viruses and in particular for the quantification of viruses within vaccine production processes for diagnostic applications.

Further useful primers are SEQ ID NOs: 12, 13, 14 and 15. Further useful probes are SEQ ID NOs: 16 and 17. SEQ ID NOs: 14 and 15 can be used in combination with SEQ ID NO: 16. SEQ ID NO 17 can be used in combination with SEQ ID NOs: 4 and 7.

Probes *(e.g.* SEQ ID NOs: 3 and 9) may be labeled *e.g.* with a 5' 6-carboxyfluorescein (6FAM) label and/or a 3' 'BlackBerry Quencher' (BBQ) label.

The disclosure also describes nucleic acids which comprise a nucleotide sequence selected from SEQ ID NOs 1, 2, 3, 4, 5, 6, 7, 8, 9, 10, or 11. These nucleic acids should be single-stranded with a length of less than 80 nucleotides *e.g.* less than 50 nucleotides, or less than 30 nucleotides. They can be useful as primers and/or probes for detecting influenza viruses. The nucleic acid may have the same 3' residue as the relevant SEQ ID NO: *i.e.* it may comprise a sequence 5'-X-Y-3' where: Y is a sequence selected from SEQ ID NOs 1, 2, 3, 4, 5, 6, 7, 8, 9, 10, and 11; and X is a nucleotide sequence of 1 or more nucleotides. The nucleic acid with sequence 5'-X-Y-3' can hybridise to an influenza virus matrix nucleic acid.

### BRIEF DESCRIPTION OF THE DRAWINGS

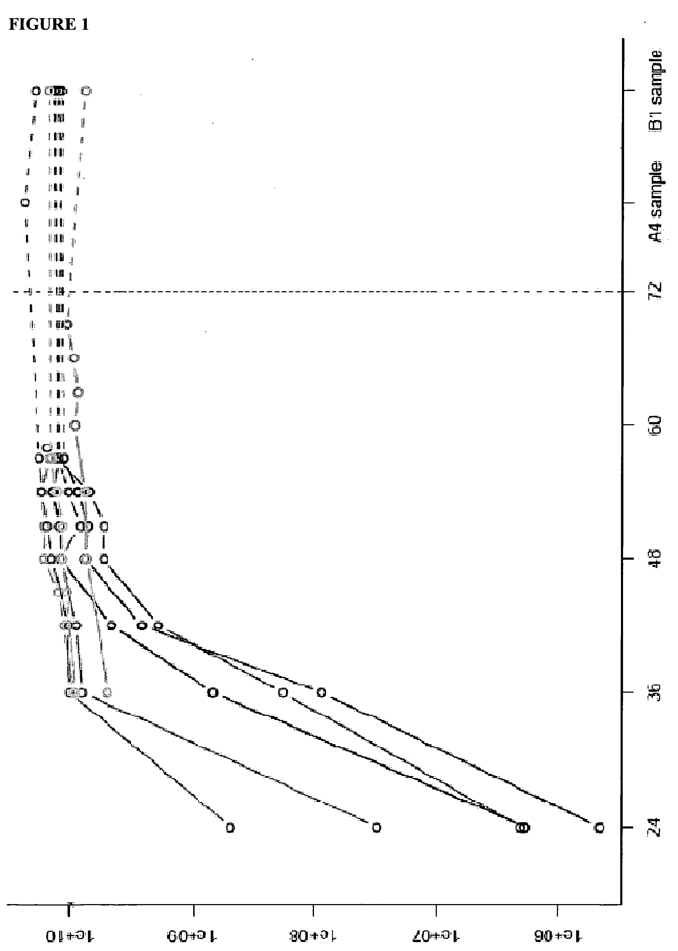

Figure 1 shows the correlation of qPCR virus particle measurements with known infectious virus kinetics, measured during infection. The x-axis indicates hours post infection. The y-axis shows the number of copies per mL as assessed by qPCR on a logarithmic scale. The figure illustrates that the curves of qPCR correspond to standard infectious virus titre kinetics at low m.o.i. In particular, virus offspring is measurable in supernatants after 16-24 hrs and maximum infectivity is seen after 48 hrs. Each line shows a different experiment.

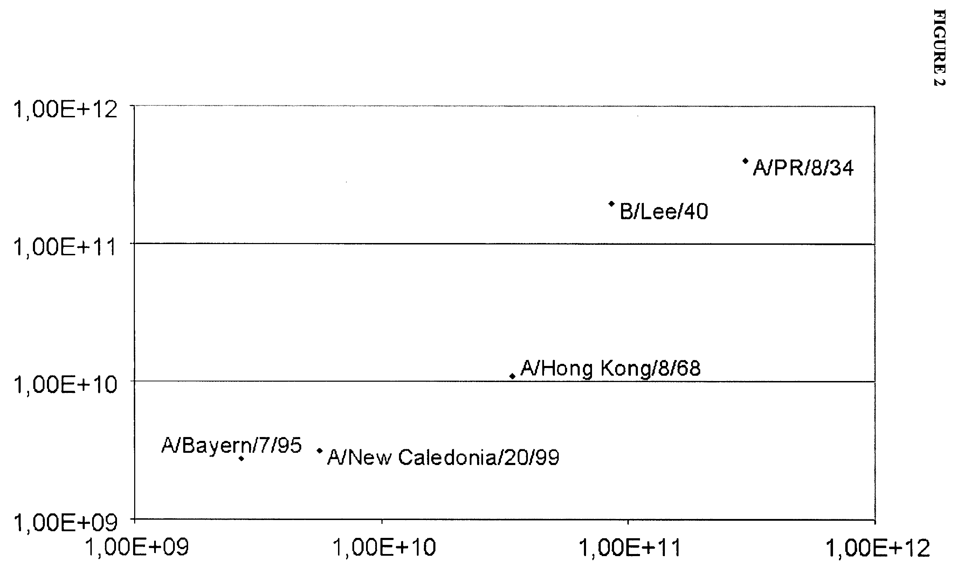

Figure 2 shows the correlation of virus particles as assessed by transmission electron microscopy versus the number of virus particles as assessed by qPCR according to the present invention. The x-axis shows the number of TEM virus particle counts per mL and the y-axis shows the number of copies per mL as assessed by qPCR.

### MODES FOR CARRYING OUT THE INVENTION

### Detection of different influenza subtypes

Influenza virus strains are obtained from the German national reference center for animal influenza (Frierich Loeffler Institute, Riems). The strains are cultured on MDCK 33016PF cells and supernatants of passage one and two are analysed using the RT-PCR methods of the invention using either a Qiagen QuantiTect™ Virus kit or an Invitrogen Super Script(TM) III Platinum™ kit. The primers and probes used are those shown in table 1.

When the QuantiTect™ Virus kit is used the following temperature profiles are run:
- measurement of fluorescence signal: 60 °C
- ramp rate 2°C/sec
- RT step: 50°C for 20 min
- Taq activation 95°C for 5 min
- Main run: 45 cycles: 94°C for 15 sec; 60°C for 45 sec (2-step process) or 45 cycles: 94°C for 15 sec; 60°C for 30 sec; 72°C for 30 sec (3-step process).

When the Super Script(tm) III Platinum^{™} kit is used the following temperature profiles are run:
- measurement of fluorescence signal: 60 °C
- ramp rate 2°C/sec
- RT step: 50°C for 15 min
- Taq activation 95°C for 2 min
- Main run: 45 cycles: 94°C for 15 sec, 60°C for 45 sec

The fluorescent signals are analysed using the respective real time software. The signals are quantified by comparing the generated fluorescent signal with the respective signal of a dilution series of an IVT. The results shown in table 2 demonstrate that all of the tested influenza virus subtypes can be identified with the same set of primer and probe. This shows that the methods of the invention are capable of detecting several different influenza subtypes. The inventors also tested the influenza strains shown in table 7.

In order to assess the sensitivity of the methods of the invention, a serial dilution of samples containing either the A/Bayern/7/95 influenza strain or the B/Baden Württemberg/3/06 influenza strain is done. The samples are subjected to qPCR using either the QuantiTect™ Virus kit or Super Script(tm) III Platinum™ kit in accordance with the methods of the present invention (as described above) or the Cepheid™ Influenza Virus A/B Primer and Probe Set following the manufacturer's protocol. The fluorescent signals are analysed using the respective real time software. The signals are quantified by comparing the generated fluorescent signal with the respective signal of a dilution series of an IVT. The results are shown in tables 5 and 6. For the influenza A strain, it is shown that virus particles are still detectable up to a dilution of 1:1000000 when using the methods of the invention wherein the Cepheid kit can detect virus particles only up to a dilution of 1:100000. For the influenza B strain, virus particles are still detectable up to a dilution of 1:1000000 wherein the Cepheid kit can detect virus particles only up to a dilution of 1:10000. The methods of the invention are therefore much more sensitive than the prior art methods.

### Quantification of influenza virus particles in sample

Influenza virus particles are measured in a sample as outlined in figure 1. Briefly, a 21µl sample is subjected to RNase using a mixture of 15U of RNase A and 7.5U of T1. The sample is incubated with the RNase mixture for one hour. The pretreated sample is diluted at ratios of 1:100 to 1:10000 in order to obtain a dilution at which qPCR gives results in a linear range. The sample is subjected to qPCR in accordance with the methods of the invention. The results are compared a standard curve using an *in vitro* transcribed RNA (IVT). The results (illustrated in Figure 1) show that the obtained curves of qPCR copy numbers correspond to standard infectious virus titre kinetics at low MOI. In particular, virus offspring is measurable in supernatants after 16-24 hours and maximum infectivity titres are observed after 48 hours.

### Correlation of virus particles as assessed by transmission electron microscopy and qPCR according to the present invention

The influenza strains obtained are A/Bayern/7/95, A/New Caledonia/20/99, A/Hong Kong/8/68, B/Lee/40 and A/Puerto Rico/8/34 are obtained from ABI online. The number of virus particles is assessed by transmission electron microscopy (TEM). To this end, virus particles are applied to coated copper grids and air dried. The material is then fixed with 2.5% (v/v) glutaraldehyde, washed and stained with 2% (w/v) aqueous uranyl acetate and 2% (w/v) phosphotungstic acid (PTA) pH 6.5. The number of virus particles in the sample is determined by TEM. The number of viruses particles counted by TEM is compared to the number of virus particles as assessed by qPCR according to the present invention. The results (see Figure 2) show that the methods of the invention accurately determine the number of virus particles in a sample for various different influenza strains.

**Table 1: preferred primer and probe sequences for the detection of influenza A and B virus. The probe sequences are Taqman™ probes.**

| **SEQ ID NO** | **Name (primer/probe)** | **Sequence** | **Genus** | **Tm°C** |
|---|---|---|---|---|
| 1 | InfBM_BR17 (primer) | gcagatagaggcaccaattagtg | B | 62.9 |
| 2 | InfA_M_BR9 (primer) | caggccccctcaaag | A | 51.6 |
| 3 | InfA_M_TMa (probe) | aggtgacaggattggtcttgtctttagcc | A | 70.4 |
| 4 | InfA_MBR11 (primer) | gcgtctacgctgcagtcc | A | 60.7 |
| 5 | InfA_MBR12 (primer) | cttctaaccgaggtcgaaacg | A | 61.3 |
| 6 | InfA_MBR13 (primer) | gaccaatcctgtcacctctgac | A | 6.0 |
| 7 | InfAM_BR18 (primer) | caggccccctcaaagc | A | 55.8 |
| 8 | InfB_M_BR8 (primer) | gtgctttytgtatatcagttargc | B | 60.1 |
| 9 | InfB_M_TM (probe) | agatggagaaggcaaagcagaactagc | B | 68.3 |
| 10 | InfB_MBR10 (primer) | gatagaggcaccaattagtg | B | 56.4 |
| 11 | InfBM_BR16 (primer) | gtttggagacacaattgcctacc | B | 62.9 |
| | | | | |
| 12 | BF (Youil) | ctgtttggagacacaattgc | B | |
| 13 | BR (Youil) | gtgctttytgtatatcag | B | |
| 14 | FluSw H1 F236 | tgggaaatccagagtgtgaatcact | A | |
| 15 | FluSw H1 R318 | cgttccattgtctgaactagrtgtt | A | |
| 16 | FluSw H1 TM292 | ccacaatgtaggaccatgagcttgctgt | A | |
| 17 | InfA_M_swine | aggtgacaagattggtcttgtctttagcc | A | |

**Table 2**

| **Subtype** | **AIV-Strain** | **HA-Test** | | **qPCR copies/ml** | |
|---|---|---|---|---|---|
| | | **Passage** | | **Passage** | |
| | | **1** | **2** | **1** | **2** |
| **H5N6** | A/duck/Potsdam/2243/84 | 128 | 64 | 2,9E+09 | 1,0E+09 |
| **H9N2** | A/turkey/Germany/176/95 | 128 | 128 | 7,9E+08 | 3.0E+08 |
| **H3** | R2076/3/07 | 64 | 64 | 6,8E+07 | 3,2E+08 |
| **H4N6** | R2619/2/07 | <2 | <2 | 6,1E+02 | <100 |
| **H6** | R2707/2/07 | <2 | <2 | 4,9E+03 | <100 |
| **H8** | R2709/2/07 | <2 | <2 | 5,1E+02 | <100 |
| **H2N1** | R2711/2/07 | <2 | <2 | 2,0E+03 | <100 |

**Table 3**

| | | | | | |
|---|---|---|---|---|---|
| A/Bangkok/1/1979(H3N (1027) | | | A/Wellington/4/2003 (977) | | |
| A/canine/Texas/1/2004(H3N8) (1026) | | | A/Canterbury/152/2001 (1000) | | |
| A/chicken/Bangli Bali/BBPV6-1/2004 (981) | | | A/Chicken/California/9420/2001(H6N2) (1002) | | |
| A/chicken/Germany/R28/03(H7N7) (982) | | | A/Chicken/Shanghai/F/98(H9N2) (1027) | | |
| A/Dk/ST/5048/2001(H3N8) (988) | | | A/duck/Hokkaido/9/99 (H9N2) (986) | | |
| A/Dunedin/10/2002 (977) | | | A/equine/Ohio/1/2003(H3N8) (1027) | | |
| A/FPV/Dobson/27 (H7N7) (1027) | | | A/Hong Kong/1073/99 (1025) | | |
| A/human/Zhejiang/16/2006(H5N1) (998) | | | A/Leningrad/134/17/57 (1027) | | |
| A/mallard/Alberta/24/01 (1027) | | | A/Memphis/15/1983 (1000) | | |
| A/New Caledonia/20/1999(H1N1) (985) | | | A/New York/719/1994 (978) | | |
| A/seal/Mass/1/80(H7N7) (1027) | | | A/swan/Mongolia/2/06(H5N1) (987) | | |
| A/swine/IN/PU542/04 (H3N1) (1030) | | | A/swine/Iowa/15/1930 (1027) | | |
| A/Thailand/1(KAN-1)/2004(H5N1) (1039) | | | A/Thailand/676/2005(H5N1) (1003) | | |
| A/USSR/90/1977(H1N1) (1027) | | | A/USSR/90/77 (1000) | | |
| A/Vietnam/CL119/2005(H5N1) (982) | | | AA/WDk/ST/1737/2000(H6N8) (990) | | |
| A/WDk/ST/988/2000(H4N9) (815) | | | | | |
| AB166865 | AB188819 | AB189048 | AB212651 | AB239305 | AF046082 |
| AF073180 | AF073181 | AF073182 | AF073185 | AF073186 | AF073187 |
| AF073188 | AF073189 | AF073190 | AF073192 | AF073193 | AF073195 |
| AF073203 | AF073205 | AF156458 | AF156459 | AF156464 | AF156466 |
| AF156467 | AF156468 | AF203788 | AF231360 | AF231361 | AF250486 |
| AF262213 | AF398876 | AF401293 | AF474049 | AF474050 | AF474051 |
| AF474052 | AF474053 | AF474054 | AF474055 | AF474056 | AF474057 |
| AF474058 | AF508687 | AF508692 | AF508693 | AF508694 | AF508695 |
| AF508696 | AF508697 | AF508698 | AF508700 | AF508702 | AY075029 |
| AY130766 | AY210030 | AY210042 | AY210047 | AY210051 | AY210053 |
| AY210054 | AY221537 | AY241600 | AY241612 | AY684709 | AY862620 |
| AY210063 | AY221538 | AY241601 | AY241613 | AY724262 | AY862621 |
| AY210065 | AY241591 | AY241602 | AY241614 | AY724263 | AY862622 |
| AY210244 | AY241592 | AY241603 | AY253755 | AY737292 | AY862623 |
| AY210253 | AY241593 | AY241604 | AY303652 | AY737298 | AY950237 |
| AY210258 | AY241594 | AY241605 | A303653 | AY770077 | AY950238 |
| AY210264 | AY241595 | AY241606 | AY303654 | AY770998 | AY950239 |
| AY210265 | AY241596 | AY241607 | AY303655 | AY800234 | AY950240 |
| AY221530 | AY241597 | AY241608 | AY303656 | AY818145 | AY950241 |
| AY221531 | AY241598 | AY241609 | AY340091 | AY862615 | CY002955 |
| AY221532 | AY241599 | AY241610 | AY590578 | AY862616 | CY004584 |
| AY221533 | AY651391 | AY241611 | AY609315 | AY862617 | CY004722 |
| AY221536 | AY651413 | CY007036 | AY611525 | CY007220 | CY005445 |
| CY005519 | AY653194 | CY007044 | AY646079 | CY007228 | CY005448 |
| CY005606 | CY006956 | CY007052 | CY007132 | CY007236 | CY007356 |
| CY005653 | CY006964 | CY007060 | CY007140 | CY007244 | CY007364 |
| CY005692 | CY006972 | CY007068 | CY007148 | CY007252 | CY007372 |
| CY005832 | CY006980 | CY007076 | CY007156 | CY007260 | CY007380 |
| CY005839 | CY006988 | CY007084 | CY007164 | CY007268 | CY007388 |
| CY006045 | CY006996 | CY007092 | CY007172 | CY007292 | CY007396 |
| CY006924 | CY007004 | CY007100 | CY007188 | CY007300 | CY007404 |
| CY006932 | CY007012 | CY007108 | CY007196 | CY007316 | CY007412 |
| CY006940 | CY007020 | CY007116 | CY007204 | CY007340 | CY007420 |
| CY006948 | CY007028 | CY007124 | CY007212 | CY007348 | CY007428 |
| CY007436 | CY007668 | CY007868 | CY008084 | CY008349 | CY008589 |
| CY007444 | CY007676 | CY007876 | CY008092 | CY008357 | CY008597 |
| CY007452 | CY007684 | CY007884 | CY008100 | CY008365 | CY008605 |
| CY007460 | CY007692 | CY007892 | CY008108 | CY008373 | CY008613 |
| CY007468 | CY007700 | CY007900 | CY008132 | CY008381 | CY008621 |
| CY007476 | CY007708 | CY007916 | CY008140 | CY008389 | CY008629 |
| CY007484 | CY007716 | CY007924 | CY008157 | CY008397 | CY008637 |
| CY007492 | CY007724 | CY007932 | CY008197 | CY008405 | CY008645 |
| CY007500 | CY007732 | CY007940 | CY008221 | CY008413 | CY008653 |
| CY007508 | CY007740 | CY007948 | CY008229 | CY008421 | CY008749 |
| CY007516 | CY007748 | CY007956 | CY008237 | CY008429 | CY008757 |
| CY007524 | CY007756 | CY007964 | CY008245 | CY008437 | CY008765 |
| CY007532 | CY007764 | CY007988 | CY008253 | CY008445 | CY008773 |
| CY007540 | CY007772 | CY007996 | CY008261 | CY008477 | CY008781 |
| CY007548 | CY007780 | CY008004 | CY008269 | CY008485 | CY008789 |
| CY007556 | CY007788 | CY008012 | CY008277 | CY008493 | CY008797 |
| CY007564 | CY007796 | CY008020 | CY008285 | CY008501 | CY008805 |
| CY007572 | CY007804 | CY008028 | CY008293 | CY008509 | CY008821 |
| CY007580 | CY007812 | CY008036 | CY008301 | CY008541 | CY008829 |
| CY007588 | CY007820 | CY008044 | CY008309 | CY008549 | CY008837 |
| CY007596 | CY007828 | CY008052 | CY008317 | CY008557 | CY008845 |
| CY007604 | CY007844 | CY008060 | CY008325 | CY008565 | CY008853 |
| CY007652 | CY007852 | CY008068 | CY008341 | CY008573 | CY008861 |
| CY007660 | CY007860 | CY008076 | CY008333 | CY008581 | CY009021 |
| CY009029 | CY009581 | CY010141 | CY010413 | CY015054 | DQ064395 |
| CY009037 | CY009589 | CY010149 | CY010421 | CY015074 | DQ064396 |
| CY009045 | CY009597 | CY010157 | CY010429 | CY015082 | DQ064397 |
| CY009077 | CY009621 | CY010165 | CY010437 | CY015097 | DQ064398 |
| CY009085 | CY009757 | CY010173 | CY010445 | CY015109 | DQ064399 |
| CY009093 | CY009765 | CY010181 | CY010453 | CY015116 | DQ064402 |
| CY009101 | CY009789 | CY010189 | CY010461 | CY015152 | DQ064403 |
| CY009109 | CY009797 | CY010197 | Cry010469 | CY015444 | DQ064404 |
| CY009117 | CY009805 | CY010205 | CY010477 | CY016125 | DQ064405 |
| CY009133 | CY009813 | CY010213 | CY010549 | CY016277 | DQ064406 |
| CY009141 | CY009821 | CY010221 | CY010557 | CY016285 | DQ064407 |
| CY009149 | CY009829 | CY010229 | CY010765 | CY016293 | DQ083661 |
| CY009157 | CY009845 | CY010237 | CY010773 | CY016301 | DQ083665 |
| CY009165 | CY009853 | CY010245 | CY010781 | DQ009919 | DQ083666 |
| CY009181 | CY009861 | CY010253 | CY011065 | DQ021745 | DQ083668 |
| CY009189 | CY009877 | CY010261 | CY011081 | DQ021746 | DQ083669 |
| CY009197 | CY009885 | CY010269 | CY011089 | DQ021758 | DQ083670 |
| CY009213 | CY009933 | CY010277 | CY011409 | DQ064381 | DQ083671 |
| CY009221 | CY009941 | CY010285 | CY011777 | DQ064382 | DQ083672 |
| CY009229 | CY009949 | CY010293 | CY011785 | DQ064383 | DQ083675 |
| CY009277 | CY009957 | CY010301 | CY012433 | DQ064384 | DQ083678 |
| CY009389 | CY009965 | CY010309 | CY013241 | DQ064385 | DQ083679 |
| CY009397 | CY009973 | CY010317 | CY014672 | DQ064386 | DQ083682 |
| CY009413 | CY009981 | CY010325 | CY014822 | DQ064387 | DQ083686 |
| CY009421 | CY010085 | CY010333 | CY014881 | DQ064388 | DQ083687 |
| CY009437 | CY010093 | CY010341 | CY014910 | DQ064389 | DQ083688 |
| CY009533 | CY010101 | CY010349 | CY015007 | DQ064390 | DQ083690 |
| CY009541 | CY010109 | CY010365 | CY015015 | DQ064391 | DQ083692 |
| CY009549 | CY010117 | CY010381 | CY015020 | DQ064392 | DQ083697 |
| CY009557 | CY010125 | CY010389 | CY015028 | DQ064393 | DQ094252 |
| CY009573 | CY010133 | CY010397 | CY015034 | DQ064394 | DQ094255 |
| DQ094256 | DQ094270 | DQ095642 | DQ320961 | DQ320999 | DQ376656 |
| DQ094257 | DQ095632 | DQ095643 | DQ320964 | DQ321000 | DQ376657 |
| DQ094258 | DQ095633 | DQ095644 | DQ320976 | DQ321003 | DQ376658 |
| DQ094259 | DQ095635 | DQ095645 | DQ320992 | DQ321006 | DQ376659 |
| DQ094260 | DQ095636 | DQ095646 | DQ320993 | DQ323678 | DQ376660 |
| DQ094261 | DQ095637 | DQ236084 | DQ320994 | DQ351858 | DQ376662 |
| DQ094262 | DQ095638 | DQ237951 | DQ320995 | DQ351859 | DQ376664 |
| DQ094263 | DQ095639 | DQ320942 | DQ320996 | DQ351860 | DQ376666 |
| DQ094264 | DQ095640 | DQ320959 | DQ320997 | DQ366333 | DQ376667 |
| DQ094265 | DQ095641 | DQ320960 | DQ320998 | DQ376655 | DQ376668 |
| DQ376669 | DQ376689 | DQ492913 | DQ492939 | DQ492979 | DQ997179 |
| DQ376670 | DQ449633 | DQ492915 | DQ492940 | DQ508828 | DQ997226 |
| DQ376671 | DQ482663 | DQ492916 | DQ492943 | DQ508836 | DQ997304 |
| DQ376672 | DQ482664 | DQ492917 | DQ492948 | DQ643985 | DQ997457 |
| DQ376673 | DQ482665 | DQ492918 | DQ492952 | DQ650660 | DQ997473 |
| DQ376674 | DQ485211 | DQ492920 | DQ492953 | DQ650664 | DQ997488 |
| DQ376675 | DQ485219 | DQ492921 | DQ492954 | DQ676831 | DQ997498 |
| DQ376676 | DQ485227 | DQ492922 | DQ492956 | DQ676835 | DQ997512 |
| DQ376677 | DQ492903 | DQ492923 | DQ492957 | DQ676839 | FLAM1A |
| DQ376678 | DQ492904 | DQ492925 | DQ492959 | DQ849018 | FLAM1M2A |
| DQ376679 | DQ492905 | DQ492926 | DQ492961 | DQ997086 | FLAM2C |
| DQ376680 | DQ492906 | DQ492927 | DQ492964 | DQ997099 | IAU49119 |
| DQ376683 | DQ492907 | DQ492928 | DQ492967 | DQ997108 | IAU65564 |
| DQ376684 | DQ492909 | DQ492929 | DQ492971 | DQ997119 | IAU65571 |
| DQ376686 | DQ492911 | DQ492930 | DQ492973 | DQ997124 | IAU65574 |
| DQ376688 | DQ492912 | DQ492937 | DQ492975 | DQ997138 | |

**Table 4:**

| | | | | | | |
|---|---|---|---|---|---|---|
| B/Aichi/5/88 (1142) | | | B/Ann Arbor/1/1986 (1155) | | | |
| BBangkok/460/03 (1074) | | | B/Barcelona/215/03 (1088) | | | |
| B/Beijing/184/93 (1096) | | | B/Bucharest/795/03 (1087) | | | |
| B/England/23/04 (1053) | | | B/Hong Kong/05/1972 (1154) | | | |
| B/Hong Kong/330/2001 (1190) | | | B/Houston/1/91 (1079) | | | |
| B/Ibaraki/2/85 (1141) | | | B/Jiangsu/10/03 (1059) | | | |
| B/Lee/40 (1191) | | | B/Los Angeles/1/02 (1076) | | | |
| B/Memphis/13/03 (1076) | | | B/Nanchang/6/96 (1076) | | | |
| B/Oslo/71/04 (1095) | | | B/Panama/45/90 (1139) | | | |
| B/Singapore/222/79 (1187) | | | B/Trento/3/02 (1077) | | | |
| B/Victoria/504/2000 (1190) | | | B/Yamagata/1/73 (1188) | | | |
| B/Yamagata/K519/2001 (1076) | | | | | | |
| AB036878 | AF100375 | AF100377 | AF100380 | AF100381 | AF100382 | AF100383 |
| AF100384 | AF100385 | AF100386 | AF100387 | AF100388 | AJ783377 | AJ783379 |
| AJ783381 | AJ783382 | AJ783383 | AJ783384 | AJ783386 | AJ783387 | AJ783388 |
| AJ783392 | AJ783393 | AJ783394 | AJ783395 | AY260941 | AY260955 | AY504613 |
| AY504621 | AY581971 | AY581982 | AY581983 | AY687399 | DQ508916 | DQ508924 |
| FLBMO | NC_002210 | | | | | |

**Table 5: A/Bayern/7/95**

| Dilution | TCID50/mL (8.1) | Cₜ | Cₜ | Cₜ | Cₜ |
|---|---|---|---|---|---|
| | | **Quantitect Virus (Field-Test)** | | **Superscript III Platinum** | **Cepheid** Influenza Virus A/B Primer and Probe Set ASR |
| | | 3-step | 2-step | | |
| undiluted | 125.892.541 | 14,26 | 13,05 | 12,43 | 15,76 |
| 1:10 | 12.589.254 | 17,49 | 16,18 | 15,96 | 18,52 |
| 1:100 | 1.258.925 | 20,70 | 19,92 | 20,29 | 22,14 |
| 1:1000 | 125.893 | 24,50 | 22,77 | 23,22 | 25,23 |
| 1:10000 | 12.589 | 27,72 | 26,55 | 26,31 | 28,96 |
| 1:100000 | 1.259 | 31,12 | 30,14 | 29,25 | 33,26 |
| 1:1000000 | 126 | 35,57 | 36,68 | 32,99 | 0,00 |

**Table 6: B/Baden-Württemberg/3/06**

| Dilution | TCID50/mL (7.9) | Cₜ | Cₜ | Cₜ | Cₜ |
|---|---|---|---|---|---|
| | | **Quantitect Virus (Field-Test)** | | **Superscript III Platinum** | **Cepheid** Influenza Virus A/B Primer and Probe Set ASR |
| | | 3-step | 2-step | | |
| Undiluted | 79.432.823 | 15,47 | 14,49 | 13,59 | 16,91 |
| 1:10 | 7.943.282 | 18,61 | 18,10 | 16,99 | 19,45 |
| 1:100 | 794.328 | 22,20 | 21,32 | 20,96 | 23,29 |
| 1:1000 | 79.433 | 25,28 | 24,27 | 24,66 | 27,10 |
| 1:10000 | 7.943 | 28,92 | 28,11 | 28,16 | 30,96 |
| 1:100000 | 794 | 0,00 | 31,54 | 31,07 | 0,00 |
| 1:1000000 | 79 | 0,00 | 36,96 | 34,77 | 0,00 |
| 1:10000000 | 8 | | | | |

**Table 7**

| | | |
|---|---|---|
| A/Bayern/7/95 | A/New Caledonia/20/99 | B/Brisbane/60/2008 |
| A/Brisbane/59/2007 IVR 148 | A/PR/8/34 | B/BadenWuerttemberg/3/2006 |
| A/Brisbane/10/2007 IVR 147 | A/Solomon Islands/03/2006 | B/Florida/04/2006 |
| A/California/04/2009 | A/LJruguay 716/07 X-175C | B/Fujian/1272/2008 |
| A/California/07/2009 X179A | A/Uruguay/716/07 | B/Lee/40 |
| A/HH/01/2009 | A/Wisconsin/67/2005 | B/Malaysia/2506/2004 |
| A/HK/8/68 | B/Bangladesh/3333/2007 | B/Perth/210/2008 |
| A/Mexiko/4108/2009 | BBrisbane/33/2008 | |

## Claims

1. A method for quantifying the amount of intact influenza virus particles in a sample, **characterised in that** the following steps are conducted:
a) free virus RNA is removed from the sample,
b) a method for detecting the remaining influenza virus RNA in the sample is applied, **characterised in that** a conserved region within the influenza genome is amplified,
c) the signal generated in part (b) is compared to the signal generated by a standard RNA,
d) the amount of intact virus particles is concluded.

2. A method according to claim 1, wherein the conserved region codes partly or completely for the M protein.

3. A method according to claim 1, wherein the method provides an amplicon comprising SEQ ID NO 3 or SEQ ID NO 9.

4. A method according to one of the preceding claims, **characterised in that** a one step RT-qPCR is conducted.

5. A method according to claim 4 **characterised in that** at least one of the primers or probes of Table 1 (SEQ ID NOs 1-11) is used.

6. The method of claim 1, wherein the RNA in step (a) is removed by RNase treatment.

7. A method for the production of influenza vaccines, in which a method described in one of the preceding claims is applied within the fermentation step of a cell culture based production process, and that the amount of virus particle is quantified in order to determine the optimal time for harvesting the viruses.

8. A method for the cell culture-based production of an influenza vaccine, **characterised in that** the following steps are conducted:
• cells are propagated in a fermentation vessel;
• seed viruses are added;
• the virus propagation is monitored using the method of any one of claims 1-6;
• the virus suspension is centrifuged and filtered;
• the virus is purified by chromatography and ultra-/diafiltration steps, inactivated, disrupted to solubilize the viral surface antigens HA and NA;
• the antigens are filtrated to obtain monovalent bulk; and
• optionally, blending the monovalent bulk into multivalent bulks, more preferably trivalent bulks, and filling into final container.

## Patentansprüche

1. Verfahren zum Quantifizieren der Menge an Influenzavirus-Partikeln in einer Probe, **dadurch gekennzeichnet, dass** die folgenden Schritte durchgeführt werden:
a) freie Virus-RNA wird aus der Probe entfernt,
b) ein Verfahren zum Nachweisen der verbleibenden Influenzavirus-RNA in der Probe wird angewendet, **dadurch gekennzeichnet, dass** eine konservierte Region innerhalb des Influenza-Genoms amplifiziert wird,
c) das in Teil (b) hervorgerufene Signal wird mit dem durch eine Standard-RNA hervorgerufenen Signal verglichen,
d) auf die Menge an intakten Viruspartikeln wird geschlossen.

2. Verfahren nach Anspruch 1, wobei die konservierte Region teilweise oder vollständig für das M-Protein codiert.

3. Verfahren nach Anspruch 1, wobei das Verfahren ein Amplikon bereitstellt, das SEQ ID NO 3 oder SEQ ID NO 9 umfasst.

4. Verfahren nach einem der vorhergehenden Ansprüche, **dadurch gekennzeichnet, dass** eine Ein-Schritt-RT-qPCR durchgeführt wird.

5. Verfahren nach Anspruch 4, **dadurch gekennzeichnet, dass** mindestens einer der Primer oder eine der Sonden von Tabelle 1 (SEQ ID NO 1-11) verwendet wird.

6. Verfahren nach Anspruch 1, wobei die RNA in Schritt (a) durch RNase-Behandlung entfernt wird.

7. Verfahren zur Herstellung von Influenza-Impfstoffen, wobei ein Verfahren nach einem der vorhergehenden Ansprüche im Fermentationsschritt eines auf Zellkultur basierenden Herstellungsverfahrens angewendet wird, und dass die Menge an Viruspartikel quantifiziert wird, um den optimalen Zeitpunkt für die Ernte der Viren zu bestimmen.

8. Verfahren zur auf Zellkultur basierenden Herstellung eines Influenza-Impfstoffs, **dadurch gekennzeichnet, dass** die folgenden Schritte durchgeführt werden:
• Zellen werden in einem Fermentationsgefäß vermehrt,
• Impf-Viren werden zugegeben,
• die Virusvermehrung wird unter Verwendung des Verfahrens nach einem der Ansprüche 1-6 überwacht,
• die Virussuspension wird zentrifugiert und filtriert,
• das Virus wird durch Chromatographie- und Ultra-/Diafiltrationsschritte gereinigt, inaktiviert, zerstört, um die viralen Oberflächenantigene HA und NA zu solubilisieren,
• die Antigene werden filtriert, um einen monovalenten Bulk zu erhalten, und
• gegebenenfalls Mischen des monovalenten Bulks zu multivalenten Bulks, stärker bevorzugt trivalenten Bulks,
und Abfüllen in den endgültigen Behälter.

## Revendications

1. Procédé pour quantifier la quantité de particules intactes du virus de la grippe dans un échantillon, **caractérisé en ce que** les étapes suivantes sont réalisées :
a) l'ARN viral libre est enlevé de l'échantillon,
b) un procédé pour détecter l'ARN résiduel du virus de la grippe est appliqué, **caractérisé en ce qu'**une région conservée dans le génome de la grippe est amplifiée,
c) le signal généré dans la partie b) est comparé au signal généré par de l'ARN témoin,
d) la quantité de particules intactes du virus est déduite.

2. Procédé selon la revendication 1, la région conservée codant partiellement ou complètement pour la protéine M.

3. Procédé selon la revendication 1, le procédé utilisant un amplicon comprenant les séquences SEQ ID NO 3 ou SEQ ID NO 9.

4. Procédé selon l'une quelconque des revendications précédentes, **caractérisé en ce qu'**une étape RT-qPCR (PCR quantitative en temps réel) est réalisée.

5. Procédé selon la revendication 4, **caractérisé en ce qu'**au moins une des amorces ou sondes du tableau 1 (SEQ ID NO 1-11) est utilisée.

6. Procédé selon la revendication 1, l'ARN dans l'étape a) étant éliminé par un traitement par ARNase.

7. Procédé pour la production de vaccins contre la grippe, dans lequel un procédé décrit dans l'une quelconque des revendications précédentes est appliqué dans l'étape de fermentation d'un procédé de production basé sur une culture cellulaire et la quantité de particule de virus est quantifiée afin de déterminer la durée optimale pour récolter les virus.

8. Procédé pour la production basé sur une culture cellulaire d'un vaccin contre la grippe, **caractérisé en ce que** les étapes suivantes sont réalisées :
- les cellules sont propagées dans une cuve de fermentation ;
- des virus d'ensemencement sont ajoutés ;
- la propagation de virus est suivie à l'aide du procédé selon l'une quelconque des revendications 1-6 ;
- la suspension de virus est centrifugée et filtrée ;
- le virus est purifié par des étapes de chromatographie et d'ultrafiltration/diafiltration, inactivé, désintégré pour solubiliser les antigènes viraux de surface HA et NA ;
- les antigènes sont filtrés pour obtenir un vrac monovalent ; et
- éventuellement, le vrac monovalent est mélangé en vrac multivalent, plus préférablement en vrac trivalent,
et transvasement dans un récipient final.


### SEQUENCE LISTING

<110> NOVARTIS AG
<120> GENERIC ASSAYS FOR DETECTION OF INFLUENZA VIRUSES
<130> P054248WO
<150> 61/215,704
   <151> 2009-05-08
<150> 61/217,045
   <151> 2009-05-26
<160> 17
<170> Seqwin99, version 1.02
<210> 1
   <211> 23
   <212> DNA
   <213> Artificial Sequence
<220>
   <223> Primer sequence
<400> 1
   gcagatagag gcaccaatta gtg 23
<210> 2
   <211> 15
   <212> DNA
   <213> Artificial Sequence
<220>
   <223> Primer sequence
<400> 2
   caggccccct caaag 15
<210> 3
   <211> 29
   <212> DNA
   <213> Artificial Sequence
<220>
   <223> Probe sequence
<400> 3
   aggtgacagg attggtcttg tctttagcc 29
<210> 4
   <211> 18
   <212> DNA
   <213> Artificial Sequence
<220>
   <223> Primer sequence
<400> 4
   gcgtctacgc tgcagtcc 18
<210> 5
   <211> 21
   <212> DNA
   <213> Artificial Sequence
<220>
   <223> Primer sequence J
<400> 5
   cttctaaccg aggtcgaaac g 21
<210> 6
   <211> 22
   <212> DNA
   <213> Artificial Sequence
<220>
   <223> Primer sequence
<400> 6
   gaccaatcct gtcacctctg ac 22
<210> 7
   <211> 16
   <212> DNA
   <213> Artificial sequence
<220>
   <223> Primer sequence
<400> 7
   caggccccct caaagc 16
<210> 8
   <211> 24
   <212> DNA
   <213> Artificial sequence
<220>
   <223> Primer sequence
<220>
   <221> misc_feature
   <222> 8
   <223> Y = C or T
<220>
   <221> misc_feature
   <222> 22
   <223> R = A or G
<400> 8
   gtgctttytg tatatcagtt argc 24
<210> 9
   <211> 27
   <212> DNA
   <213> Artificial sequence
<220>
   <223> Probe sequence
<400> 9
   agatggagaa ggcaaagcag aactagc 27
<210> 10
   <211> 20
   <212> DNA
   <213> Artificial sequence
<220>
   <223> Primer sequence
<400> 10
   gatagaggca ccaattagtg 20
<210> 11
   <211> 23
   <212> DNA
   <213> Artificial sequence
<220>
   <223> Primer sequence
<400> 11
   gtttggagac acaattgcct acc 23
<210> 12
   <211> 20
   <212> DNA
   <213> Primer sequence
<400> 12
   ctgtttggag acacaattgc 20
<210> 13
   <211> 18
   <212> DNA
   <213> Artificial Sequence
<220>
   <223> Primer sequence
<220>
   <221> misc_feature
   <222> 8
   <223> Y = C or T
<400> 13
   gtgctttytg tatatcag 18
<210> 14
   <211> 25
   <212> DNA
   <213> Artificial Sequence
<220>
   <223> Primer sequence
<400> 14
   tgggaaatcc agagtgtgaa tcact 25
<210> 15
   <211> 25
   <212> DNA
   <213> Artificial Sequence
<220>
   <223> Primer sequence
<220>
   <221> misc_feature
   <222> 21
   <223> R = A or G
<400> 15
   cgttccattg tctgaactag rtgtt 25
<210> 16
   <211> 28
   <212> DNA
   <213> Artificial Sequence
<220>
   <223> Probe sequence
<400> 16
   ccacaatgta ggaccatgag cttgctgt 28
<210> 17
   <211> 29
   <212> DNA
   <213> Artificial Sequence
<220>
   <223> Primer sequence
<400> 17
   aggtgacaag attggtcttg tctttagcc 29